# EUROPÄISCHE PATENTSCHRIFT

(11) **EP 0 957 803 B1**
(45) Veröffentlichungstag und Bekanntmachung des Hinweises auf die Patenterteilung: **31.03.2004**
(21) Anmeldenummer: 97953883.2
(22) Anmeldetag: 19.12.1997
(51) Int. Cl.: A61B 19/00

(54) **VORRICHTUNG ZUM POSITIONIEREN UND FÜHREN EINES CHIRURGISCHEN WERKZEUGES BEI ORTHOPÄDISCHEN EINGRIFFEN**
DEVICE FOR POSITIONING AND GUIDING A SURGICAL INSTRUMENT DURING ORTHOPAEDIC INTERVENTIONS
DISPOSITIF PERMETTANT DE POSITIONNER ET DE DIRIGER UN INSTRUMENT CHIRURGICAL LORS D'INTERVENTIONS ORTHOPEDIQUES

(30) Priorität: 21.12.1996 DE 19653966
(43) Veröffentlichungstag der Anmeldung: 24.11.1999
(73) Patentinhaber: DEPUY INTERNATIONAL LIMITED, Beeston, Leeds LS11 8DT (GB)
(72) Erfinder: DEPUY INTERNATIONAL LIMITED, Beeston, Leeds LS11 8DT (GB)
(74) Vertreter: Albrecht, Rainer Harald, Dr.-Ing.
(86) Internationale Anmeldenummer: PCT/EP1997/007186
(87) Internationale Veröffentlichungsnummer: WO 1998/027887

(56) Entgegenhaltungen:
- EP-A- 0 456 103
- EP-A- 0 469 966
- DE-A- 19 601 857
- PREISING B ET AL: "A LITERATURE REVIEW: ROBOTS IN MEDICINE" IEEE ENGINEERING IN MEDICINE AND BIOLOGY MAGAZINE, Bd. 10, Nr. 2, 1.Juni 1991, NEW YORK, US, Seiten 13-22, XP000226911

## Beschreibung

Die Erfindung betrifft eine Vorrichtung zum Positionieren und Führen eines chirurgischen Werkzeuges bei orthopädischen Eingriffen, mit
einem Industrieroboter, der einen programmgesteuerten, mehrgelenkigen Roboterarm mit endseitiger Montageplatte aufweist,
einem an der Montageplatte gehaltenen Werkzeug für den chirurgischen Eingriff,
einer Programmsteuerung mit Rechner und
einer Sensoreinrichtung zum Abtasten von Referenzpunkten an Knochen des Patienten,
wobei in den Gelenken des Roboterarmes Sensoren zur Erfassung der Gelenkstellung in einem in der Programmsteuerung definierten Werkzeug-Koordinatensystem angeordnet sind und wobei der Roboterarm mittels der Programmsteuerung in eine Arbeitsposition für den chirurgischen Eingriff bewegbar ist.

Eine Vorrichtung mit den vorstehend beschriebenen Merkmalen ist aus EP-A 0 456 103 bekannt. Zur Objektvermessung wird das chirurgische Werkzeug durch ein Tastelement ersetzt und wird der Roboterarm an den Patienten herangeführt, indem die Servoantriebe des Roboterarmes von einem Handgerät aus oder durch Drucksensoren, die bei einer manuellen Führung des Roboterarmes ansprechen, betätigt werden. Da die Bewegungen motorisch gesteuert sind, müssen sie sehr langsam ausgeführt werden, um eine Gefährdung des Patienten auszuschließen. Das Abtasten der Referenzpunkte nimmt folglich erhebliche Zeit in Anspruch, was sich auf die Operationsdauer nachteilig niederschlägt.

Eine ähnliche Vorrichtung ist aus dem Artikel "Robodoc" fräst Knochen auf 0,1 mm genau", von Ruth Henke, erschienen in den VDI-Nachrichten, Ausgabe Nr. 30 vom 29. Juli 1994, bekannt und wird bei Hüftoperationen eingesetzt. Vor der Operation werden dem Patienten zunächst drei Schrauben am Knie und am Oberschenkelknochen eingepflanzt. Die Schrauben haben eine kleine Vertiefung, in die der Kopf eines am Roboterarm angeordneten Fühlers passt. Eine ebenfalls präoperativ durchgeführte Computertomographie liefert für die Programmsteuerung Erforderliche Patientendaten. Während des chirurgischen Eingriffes ist der Oberschenkel des Patienten in einen sterilen, mit dem Fuß des Industrieroboters starr verbundenen Haltearm eingespannt. Ein an den Knochen angelegter Messarm erfasst etwaige Lageänderungen. Zunächst führt der Chirurg den Roboterarm mit seinem endseitigen Fühler an die durch die Vertiefung der Schrauben vorgegebenen Referenzpunkte, deren Koordinaten mit den Daten der Computertomographie verglichen werden. Aus den Daten ermittelt der Rechner dann die Arbeitsposition des Roboterarmes für den chirurgischen Eingriff. Ist die Arbeitsposition ermittelt, wird der Fühler durch ein Werkzeug ersetzt und der Oberschenkelschaft bei entsprechender Vorschubbewegung des Roboterarmes programmgesteuert gefräst. Anschließend kann der Chirurg eine vorher ausgewählte Prothese einsetzen.

Die Anwendung der bekannten Vorrichtung erfordert umfangreiche präoperative Planungen unter Einsatz von Computertomographen, die nicht nur aufwändig sind, sondern auch die Operationszeit insgesamt in störendem Maße verlängern. Nachteilig ist ferner, dass der Chirurg keine Möglichkeit hat, auf den programmgesteuerten Ablauf der Operation Einfluss zu nehmen. Bei Lageänderungen des Knochen und/oder bei Nichteinhaltung vorgegebener Toleranzen unterbricht die Vorrichtung aus Sicherheitsgründen sofort ihre Arbeit.

Der Erfindung liegt die Aufgabe zu Grunde, eine Vorrichtung der eingangs beschriebenen Art anzugeben, mit der die Arbeitsposition des chirurgischen Werkzeuges schnell und exakt festgelegt werden kann. Die Vorrichtung soll intraoperativ einsetzbar sein und so ausgebildet sein, dass der Chirurg auf die Operation Einfluss nehmen kann.

Die Aufgabe wird erfindungsgemäß durch die nebengeordneten Patentansprüche 1 und 3 gelöst. Das gemeinsame Lösungsprinzip besteht, bei unterschiedlicher gerätetechnischer Ausführung, darin, dass eine unabhängig von der Stellung des Roboterarmes manuell führbare Sensoreinrichtung für eine dreidimensionale Objektvermessung in dem Werkzeug-Koordinatensystem des Industrieroboters vorgesehen ist, wobei mittels der Sensoreinrichtung bei einer von der Programmsteuerung vorgegebenen und vorher angefahrenen Grundstellung des Roboterarmes Referenzpunkte am Knochen des Patienten abtastbar sind, wobei der Rechner aus den auf das Werkzeug-Koordinatensystem bezogenen Koordinaten der Referenzpunkte die Arbeitsposition des Roboterarmes ermittelt. Es versteht sich, dass die Arbeitsposition des Roboterarmes auch eine Abfolge von Roboterarmpositionen umfassen kann, die angefahren werden müssen, um das an der Montageplatte angeordnete Werkzeug für die Durchführung des chirurgischen Eingriffes optimal zu führen und zu positionieren. Unabhängig von der gerätetechnischen Ausführung der Sensoreinrichtung ist von erfindungswesentlicher Bedeutung, dass durch eine Kopplung zwischen Sensoreinrichtung und Roboterarm, die auf unterschiedliche und im Folgenden noch näher erläuterte Weise ausgeführt sein kann, die Koordinaten der Referenzpunkte in dem Werkzeug-Koordinatensystem des Industrieroboters ermittelt werden, welches in der Programmsteuerung definiert ist.

Die Sensoreinrichtung ermöglicht es, eine für die Errechnung der Arbeitsposition des Roboterarmes ausreichende Anzahl gut identifizierbarer Referenzpunkte an den von dem chirurgischen Eingriff betroffenen Knochen zu erfassen. Während der Aufnahme dieser Referenzpunkte bleibt der Roboterarm in der zuvor angefahrenen, bekannten Grundstellung stehen. Mit den Koordinaten der Grundstellung und den Koordinaten der Referenzpunkte kann die Arbeitsposition, die der Roboterarm anschließend des chirurgischen Eingriffes einnimmt, exakt errechnet werden. Abhängig von der Art des chirurgischen Eingriffes wird eine einzige Arbeitsposition oder eine Abfolge von Arbeitspositionen ermittelt. Die erfindungsgemäße Vorrichtung ermöglicht eine intraoperative Anwendung. Präoperative Planungen beispielsweise auf Basis von Röntgenaufnahmen oder Computertomogrammen sind prinzipiell nicht erforderlich. Ferner kann die Programmsteuerung so eingerichtet werden, das der Chirurg während der Operation an Knochen, die im Zuge der Operation freigelegt werden, mit Hilfe der Sensoreneinrichtung weitere charakteristische Punkte erfasst, die dann zur Korrektur der Arbeitsposition des Roboterarmes und/oder zur Festlegung einer Abfolge von Arbeitspositionen, die der Roboterarm bei fortschreitender Operation nacheinander einnimmt, verarbeitet werden.

Gemäß der Erfindung ist es möglich, Sensoreinrichtungen einzusetzen, die auf verschiedenen Messprinzipen beruhen. Eine Ausführungsform besteht darin, dass an die Montageplatte des Roboterarmes ein mehrgelenkiger, manuell bewegbarer Tastarm angeschlossen ist. Die Kopplung zum Werkzeug-Koordinatensystem des Roboters erfolgt in diesem Fall durch eine starre mechanische Verbindung, wobei der Befestigungspunkt des Tastarmes an der Montageplatte exakt an einer genau festgelegten Position erfolgen muss. Der Tastarm weist Gelenke mit Sensoren zur Erfassung der Gelenkstellung auf. Durch manuelle Führung des Tastarmes bei einer von der Programmsteuerung vorgegebenen und vorher angefahrenen Grundstellung des Roboterarmes sind Referenzpunkte am Knochen des Patienten abtastbar, deren Koordinaten dem Rechner zugeführt werden. Der Rechner ermittelt aus diesen Koordinaten die Arbeitsposition des Roboterarmes. Bevorzugt ist ein Tastarm mit sechs Gelenken.

Mehrgelenkige Tastarme mit Sensoren zur Erfassung der Gelenkstellungen sind an sich bekannt. Sie stellen passive, ausschließlich manuell fühlbare Messinstrumente dar, die im Rahmen der bekannten Maßnahmen als transportable Geräte zur Vermessung von Werkstücken eingesetzt werden. Die Erfindung setzt ein mit der Erkenntnis, dass der endseitige Anschluss eines mehrgelenkigen Testarmes an einem programmgesteuerten aktiven Roboterarm beachtliche Vorteil mit sich bringt, die bei chirurgischen Eingriffen genutzt werden können. Von erfindungswesentlicher Bedeutung ist, dass der an der Montageplatte angeordnete Tastarm nicht durch Werkzeuge ersetzt wird, sondern dem Chirurgen während des gesamten chirurgischen Eingriffes zur Verfügung steht.

Eine weitere Ausführung der Erfindung sieht vor, dass die Sensoreinrichtung aus einen mehrgelenkigen, an den Rechner angeschlossenen Tastarm besteht, der stationär so angeordnet ist, dass sich in dem mit dem Tastarm abgreifbaren Arbeitsraum sowohl der abzutastende Knochen des Patienten als auch die Montageplatte des in eine Grundstellung bewegten Roboterarmes befinden, wobei mit Hilfe des Tastarmes mindestens ein Referenzpunkt an der Montageplatte abtastbar ist, wobei mittels des Tastarmes sowohl die Position und Ausrichtung der Montageplatte als auch die am Knochen des Patienten abgetasteten Referenzpunkte in einem raumfesten, dreidimensionalen Koordinatensystem ermittelt werden und wobei der Rechner die ermittelten Raumkoordinaten in die Koordination des Werkzeug-Koordinatensystems transformiert. An der Montageplatte können mehrere Punkte vorgesehen sein, deren Lage genau definiert ist und in Bezug auf das Werkzeug-Koordinatensystem festliegt. Eine andere Möglichkeit besteht darin, den Roboterarm nicht nur in eine, sondern nacheinander in mehrere, zumindest drei, Grundstellungen zu bewegen. In jeder dieser Grundstellungen wird jeweils nur ein auf der Montageplatte definierter Punkt mit dem Tastarm abgetastet. Im Raum-Koordinatensystem der Tasteinrichtung hat dieser Punkt für jede der Grundstellungen des Roboterarms dann unterschiedliche Koordinaten. In Kenntnis dieser Koordinaten können die anschließend abgetasteten Raumkoordinaten der Referenzpunkte am Knochen in das Werkzeug-Koordinatensystem transformiert werden.

Eine weitere Möglichkeit zur Ausführung der Sensoreneinrichtung sieht vor, ein dreidimensionales Lokalisierungssystem einzusetzen, das eine Ortung von Gegenständen durch die Wechselwirkung von Sender/Empfängerelementen durchführt. Solche Systeme sind im Prinzip bekannt und können auf optischen, induktiven oder Ultraschallverfahren basieren. Erfindungsgemäß besteht die Sensoreinrichtung in diesem Falle aus einer Messanordnung mit Datenfernübertragung, die einen stationären Empfänger, einen Taststift mit einem ersten Sender sowie einen an der Montageplatte angeordneten zweiten Sender aufweist, wobei die Sender mehrere Signalgeber in einer fest vorgegebenen räumlichen Zuordnung aufweisen, die von dem Empfänger aufgenommene Ortungssignale abgeben, wobei aus den Ortungssignalen die Position und Ausrichtung des Taststiftes sowie der Montageplatte in einem raumfesten, dreidimensionalen Koordinatensystem ermittelt werden und wobei die ermittelten Raumkoordinaten des Taststiftes sowie der Montageplatte dem Rechner zuführbar sind, der die Raumkoordinaten des Taststiftes in die Koordinaten des Werkzeug-Koordinatensystems transformiert.

Schließlich besteht im Rahmen der Erfindung die Möglichkeit, als Sensoreinrichtung eine Messanordnung mit Datenfernübertragung einzusetzen, die einen stationären Empfänger sowie einen Taststift mit einem Sender aufweist, wobei der Sender mehrere Signalgeber in einer fest vorgegebenen räumlichen Zuordnung aufweist, die von dem Empfänger aufgenommene Ortungssignale abgeben, wobei mit Hilfe des Taststiftes mindestens ein Referenzpunkt an der Montageplatte des in eine Grundstellung bewegten Roboterarmes abtastbar ist, wobei mittels des Taststiftes sowohl die Position und Ausrichtung der Montageplatte als auch die am Knochen des Patienten abgetasteten Referenzpunkte in einem raumfesten, dreidimensionalen Koordinatensystem ermittelt werden und wobei der Rechner die ermittelten Raumkoordinaten in die Koordinaten des Werkzeug-Koordinatensystems transformiert.

Im Rahmen der Erfindung liegt es, dass der Rechner aus den Koordinaten der mit dem Tastarm oder dem Lokalisierungssystem aufgenommenen Referenzpunkte ein ablauffähiges Roboterprogramm entwickelt, das in die Programmsteuerung überspielt wird, wobei die Knochenbearbeitung programmgesteuert durch eine Bewegung des Roboterarmes erfolgt. Gemäß einer bevorzugten Ausführung der Erfindung ist das Werkzeug für den chirurgischen Eingriff jedoch auf einem ebenfalls an der Montageplatte befestigten, vorzugsweise orthogonal zur Montageplatte ausgerichteten Schlitten axial beweglich geführt. Bei dieser Ausführung der Erfindung ist die Vorschubbewegung des chirurgischen Werkzeuges manuell oder durch einen motorischen Vorschubantrieb unabhängig von der Bewegung des Roboterarmes durch den Chirurgen ausführbar, nachdem des chirurgische Werkzeug durch die erfindungsgemäße Vorrichtung in eine für die Operation optimale Lage und optimale Ausrichtung gebracht worden ist. Diese Ausführung der Erfindung ermöglicht eine kurze Operationszeit, wobei die auszuführenden Schnitte nichtsdestoweniger sehr präzise ausgeführt werden können, weil das Werkzeug mittels der erfindungsgemäßen Vorrichtung optimal gehalten und geführt wird.

In weiterer Ausgestaltung lehrt die Erfindung, dass die Vorrichtung eine zusätzliche Überwachungseinrichtung zur Erfassung der räumlichen Position des Patienten während der Operation aufweist. Die technische Ausgestaltung dieser Überwachungseinrichtung ist abhängig von dem verwendeten Lokalisierungssystem. Bei der Ausführung mit einem Tastarm wird eine Kamera eingesetzt, welche die Position von Markierungsstiften erfasst, die vor dem chirurgischen Eingriff in den betreffenden Knochen eingesetzt worden sind und deren Koordinaten mit Hilfe des Tastarmes bei der Einrichtung der Arbeitsposition des Roboterarmes abgetastet werden, wobei von der Kamera erfasste Positionsänderungen der Markierungsstifte im Rechner verarbeitet werden und durch die Programmsteuerung eine Korrektur der Arbeitsposition des Roboterarmes erfolgt. Bei Einsatz einer Sensoreinrichtung mit Sender- und Empfängerelementen weist die Überwachungseinrichtung vorzugsweise mindestens einen Referenzstift auf, der vor dem chirurgischen Eingriff an dem betreffenden Knochen des Patienten befestigbar ist und mit einem dritten Sender ausgerüstet ist, wobei der Sender des Referenzstiftes in entsprechender Weise wie der Taststift mit mehreren Signalgebern ausgerüstet ist, die von dem Empfänger aufgenommene Ortungssignale abgeben. Der Knochen kann, exakt geortet und seine Position genau berechnet werden. Bei einer Lageänderung des Knochens erfolgt eine automatische Positionskorrektur des Roboterarmes und des an dem Roboterarm angeschlossenen chirurgischen Werkzeuges, wobei gegebenenfalls eine Freigabe durch den Chirurgen erfolgen muss.

Im Folgenden wird die Erfindung anhand einer lediglich ein Ausführungsbeispiel darstellenden Zeichnung erläutert. Es zeigen schematisch
- Fig. 1: ausschnittsweise eine Draufsicht auf eine Vorrichtung zum Positionieren und Führen eines chirurgischen Werkzeuges bei orthopädischen Eingriffen,
- Fig. 2: die Seitenansicht A in Fig. 1,
- Fig. 3: die Seitenansicht B in Fig. 1 und
- Fig. 4 und 5: weitere Ausführungsformen der erfindungsgemäßen Vorrichtung.

Zum grundsätzlichen Aufbau der Vorrichtung gehören ein Industrieroboter, der einen programmgesteuerten, mehrgelenkigen Roboterarm mit endseitiger Montageplatte aufweist, sowie eine Programmsteuerung mit Rechner. In den Gelenken des Roboterarmes sind Sensoren zur Erfassung der Gelenkstellung angeordnet. Mittels der Programmsteuerung ist der Roboterarm in eine Arbeitsposition für den chirurgischen Eingriff bewegbar.

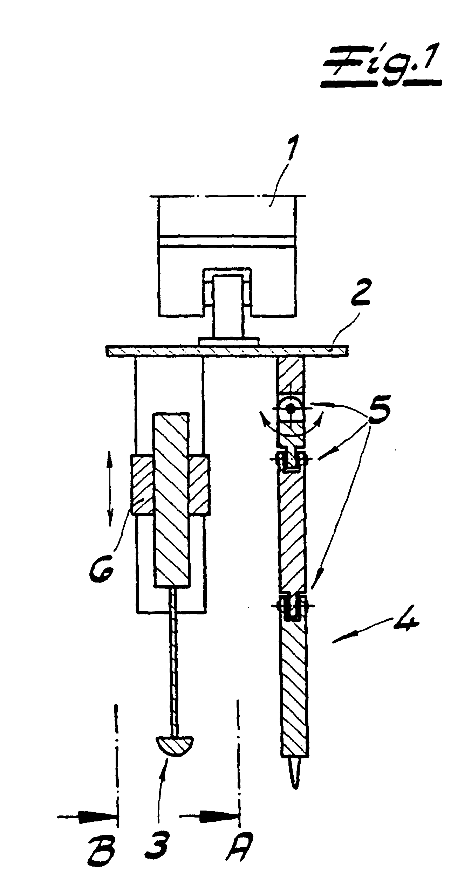

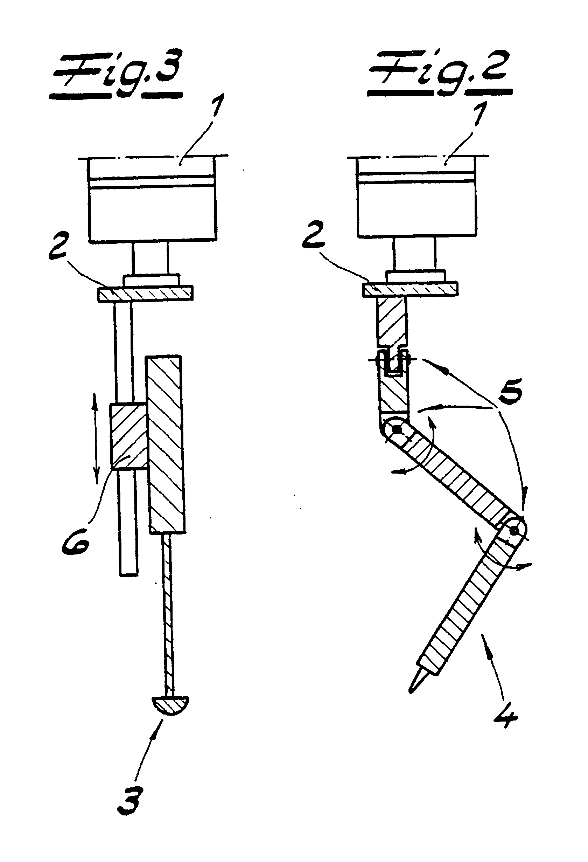

In den Fig. 1 bis 3 ist nur das freie Ende des Roboterarmes 1 dargestellt. Er weist eine endseitige Montageplatte 2 auf, an der ein Werkzeug 3 für den orthopädischen Eingriff sowie ein mehrgelenkiger, manuell bewegbarer Tastarm 4 angeschlossen sind. Einer vergleichenden Betrachtung der Fig. 1 und 2 entnimmt man, dass der Tastarm mindestens drei Gelenke 5 aufweist. Vorzugsweise sind sechs Gelenke vorgesehen. In den Gelenken 5 sitzen Sensoren, zum Beispiel in Form von Potentiometern, Inkrementalgeber, Resolvern und dergleichen, zur Erfassung der Gelenkstellungen. Durch manuelle Führung des Tastarmes 4 bei einer von der Programmsteuerung vorgegebenen Grundstellung des Roboterarmes 1 sind Referenzpunkte an Knochen des Patienten abtastbar, deren Koordinaten dem Rechner (nicht dargestellt) zugeführt werden. Aus den Koordinaten ermittelt der Rechner die Arbeitsposition, die der Roboterarm 1 anschließend während des chirurgischen Eingriffes einnimmt.

Einer vergleichenden Betrachtung der Fig. 1 und 3 entnimmt man, dass das Werkzeug 3 auf einem ebenfalls an der Montageplatte 2 befestigten Schlitten 6 axial beweglich geführt ist. Der Schlitten 6 ist orthogonal zur Montageplatte 2 angeordnet. Die Vorschubbewegung des Werkzeuges 3 ist manuell oder durch einen motorischen Vorschubantrieb unabhängig von der Bewegung des Roboterarmes 1 ausführbar.

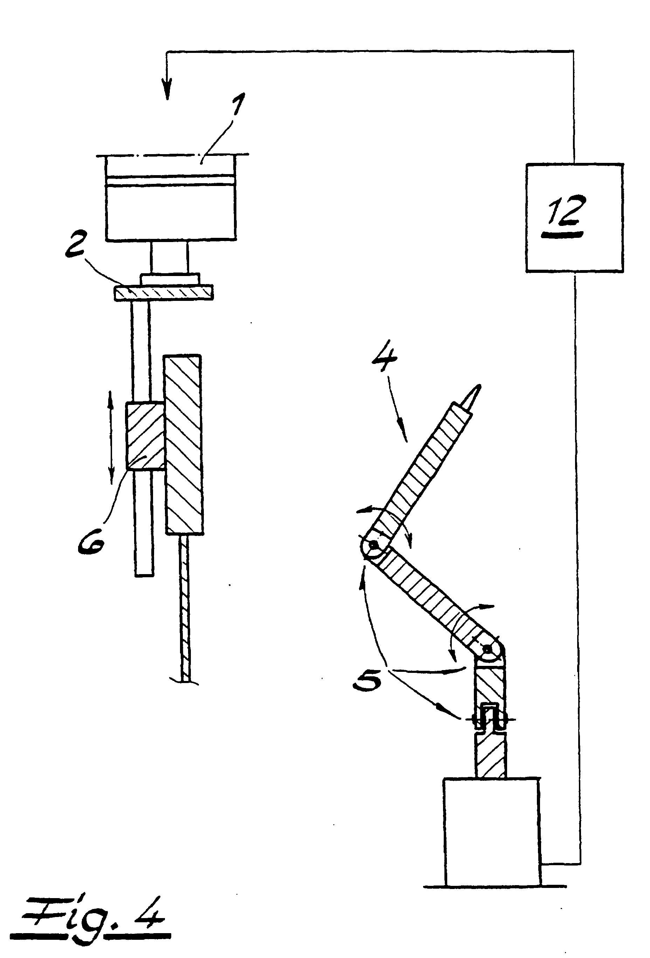

Bei der in Fig. 4 dargestellten Ausführung der erfindungsgemäßen Vorrichtung ist der Tastarm 4 nicht an der Montageplatte 2 des Roboterarmes 1 angeordnet, sondern stationär positioniert und an den Rechner 12 angeschlossen Die Position des mehrgelenkigen Tastarmes 4 ist so gewählt, dass sich in dem mit dem Tastarm 4 abgreifbaren Arbeitsraum sowohl der abzutastende Knochen als auch die Montageplatte 2 des in eine Grundstellung bewegten Roboterarmes 1 befinden. Mit Hilfe des Tastarmes ist mindestens ein Referenzpunkt an der Montageplatte 2 abtastbar. Mittels des Tastarmes 4 werden sowohl die Position und Ausrichtung der Montageplatte 2 als auch die am Knochen des Patienten abgetasteten Referenzpunkte in einem raumfesten, dreidimensionalen Koordinatensystem ermittelt. Der Rechner 12 transformiert die ermittelten Raumkoordinaten in die Koordinaten des Werkzeug-Koordinatensystems. Das Verfahren kann so durchgeführt werden, dass mit Hilfe des Tastarmes 5 mehrere Punkte an der Montageplatte 2 abgetastet werden, deren Lage in Bezug auf das Werkzeug-Koordinatensystem genau definiert sind. Ein anderes Verfahren besteht darin, den Roboterarm 1 nicht nur in eine, sondern nacheinander in mindestens drei Grundstellungen zu fahren. In jeder dieser Grundstellungen wird jeweils nur ein Punkt mit genau bekannter und definierter Position auf der Montageplatte 2 mit dem Tastarm 4 angetastet. Im Raum-Koordinatensystem der Tasteinrichtung hat dieser Punkt für jede der Grundstellungen des Roboterarmes 1 dann unterschiedliche Koordinaten. Es ist wiederum möglich, aus diesen Koordinaten eine mathematische Vorschrift zur Beschreibung der gewünschten Koordinatentransformation abzuleiten. Das Antasten der Referenzpunkte am Knochen erfolgt erst, nachdem diese mathematische Vorschrift ermittelt wurde.

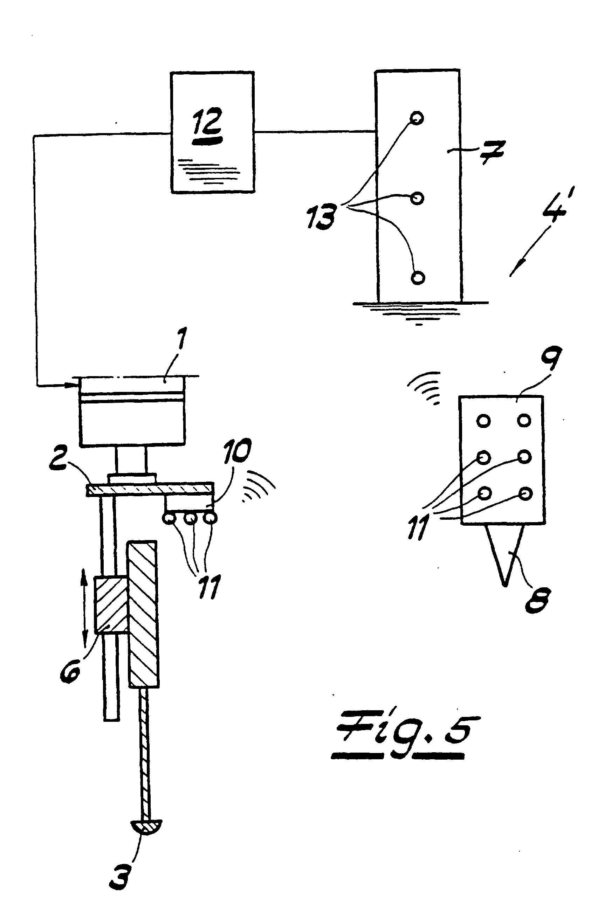

Die in Fig. 5 dargestellte Ausführung der erfindungsgemäßen Vorrichtung weist eine manuell führbare Sensoreinrichtung für eine dreidimensionale Objektvermessung in dem Werkzeug-Koordinatensystem des Roboterarmes auf, die aus einer Messanordnung 4' mit Datenfernübertragung besteht. Sie umfasst einen stationären Empfänger 7, einen Taststift 8 mit einem ersten Sender 9 sowie einen an der Montageplatte 2 angeordneten zweiten Sensor 10. Die Sender 9, 10 weisen mehrere Signalgeber 11 in einer fest vorgegebenen räumlichen Zuordnung auf, die von dem Empfänger 7 aufgenommene Ortungssignale abgeben. Aus den Ortungssignalen wird die Position und Ausrichtung des Taststiftes 8 sowie der Montageplatte 2 in einem raumfesten, dreidimensionalen Koordinatensystem ermittelt. Die ermittelten Raumkoordinaten des Taststiftes 8 sowie der Montageplatte 2 sind dem Rechner 12 zuführbar, und dieser transformiert die Raumkoordinaten des Taststiftes in die Koordinaten eines Werkzeug-Koordinatensystems, das der Programmsteuerung zu Grunde liegt.

Lokalisierungssysteme, die eine Messanordnung mit Datenfernübertragung aufweisen, sind im Prinzip bekannt und können auf optischen, induktiven oder Ultraschallverfahren basieren. Der Taststift weist beispielsweise eine kleine Platte auf, in deren Eckpunkte vier oder sechs Infrarot-Leuchtdioden 11 als Signalgeber angeordnet sind. Die von diesen ausgesendeten Lichtimpulse werden von dem stationär im Raum angeordneten Empfänger 7 erfasst, der über drei separate Empfangselemente 13, bestehend aus Optiken und lichtempfindlichen Sensoren, verfügt. Unter Ausnutzung der genau bekannten geometrischen Anordnung der Sende- sowie der Empfangselemente 11, 13 kann durch mathematische Auswertung aller Empfangssignale die Position und Orientierung des Taststiftes 8 in dem raumfesten Koordinatensystem errechnet werden. Erfindungsgemäß ist auch an der Montageplatte 2 des Roboterarmes 1 eine entsprechende kleine Platte 10 mit Sendeelementen 11 befestigt. Auf diese Weise werden sowohl der Taststift 8 als auch die Montageplatte 2 geortet, so dass durch mathematische Umrechnungen die Raumkoordinaten der mit dem Taststift ertasteten Referenzpunkten am Knochen in das der Programmsteuerung zu Grunde liegende Werkzeug-Koordinatensystem des Industrieroboters transformiert werden können.

## Patentansprüche

1. Vorrichtung zum Positionieren und Führen eines chirurgischen Werkzeuges bei orthopädischen Eingriffen, - mit
einem Industrieroboter, der einen programmgesteuerten, mehrgelenkigen Roboterarm (1) mit endseitiger Montageplatte (2) aufweist,
einem an der Montageplatte (2) gehaltenen Werkzeug (3) für den chirurgischen Eingriff,
einer Programmsteuerung mit Rechner (12) und
einer Sensoreinrichtung zum Abtasten von Referenzpunkten an Knochen des Patienten,
wobei in den Gelenken des Roboterarmes (1) Sensoren zur Erfassung der Gelenkstellung in einem in der Programmsteuerung definierten Werkzeug-Koordinatensystem angeordnet sind und wobei der Roboterarm (1) mittels der Programmsteuerung in eine Arbeitsposition für den chirurgischen Eingriff bewegbar ist, **dadurch gekennzeichnet, dass** die Sensoreinrichtung aus einem mehrgelenkigen Tastarm (4) besteht, der als passives, manuell führbares Messinstrument ausgeführt ist, Sensoren zur Erfassung der Gelenkstellungen aufweist und dem Chirurgen auch während des Eingriffes zur Verfügung steht,
wobei durch Bewegung des Tastarmes (4) die Referenzpunkte am Knochen des Patienten abtastbar sind,
während sich der Roboterarm (1) in eine von der Robotersteuerung vorgegebenen und vorher angefahrenen Grundstellung befindet und
wobei der Tastarm (4) entweder
a) an der endseitigen Montageplatte (2) des Roboterarmes (1) befestigt ist und der Rechner (12) aus den von den Sensoren des Tastarms (4) abgegebenen Positionsangaben und den im Werkzeug-Koordinatensystem gemessenen Positionsangaben der Roboterarmsensoren die Arbeitsposition des Roboterarmes (1) für den chirurgischen Eingriff ermittelt oder
b) der Tastarm (4) stationär so angeordnet ist, dass sich in dem mit dem Tastarm (4) abgreifbaren Arbeitsraum sowohl der abzutastende Knochen des Patienten als auch die Montageplatte (2) des in die Grundstellung bewegten Roboterarmes (1) befinden, und der Rechner (12) die mit dem Tastarm (4) in einem raumfesten, dreidimensionalen Koordinatensystem erfassten Koordinaten der Referenzpunkte in die Koordinaten des Werkzeug-Koordinatensystems transformiert.

2. Vorrichtung nach Anspruch 1, **gekennzeichnet durch** eine zusätzliche Überwachungseinrichtung zur Erfassung der räumlichen Position des Patienten während der Operation mit einer Kamera, welche die Position von Markierungsstiften erfasst, die vor dem chirurgischen Eingriff in den betreffenden Knochen eingesetzt worden sind und deren Koordinaten mit Hilfe des Tastarmes (4) bei der Einrichtung der Arbeitsposition des Roboterarmes (1) abtastbar sind, wobei von der Kamera erfasste Positionsänderungen der Markierungsstifte im Rechner verarbeitet werden und **durch** die Programmsteuerung eine Korrektur der Arbeitsposition des Roboterarmes (1) erfolgt.

3. Vorrichtung zum Positionieren und Führen eines chirurgischen Werkzeuges bei orthopädischen Eingriffen, - mit
einem Industrieroboter, der einen programmgesteuerten, mehrgelenkigen Roboterarm (1) mit endseitiger Montageplatte (2) aufweist,
einem an der Montageplatte (2) gehaltenen Werkzeug (3) für den chirurgischen Eingriff,
einer Programmsteuerung mit Rechner (12) und
einer Sensoreinrichtung zum Abtasten von Referenzpunkten an Knochen des Patienten,
wobei in den Gelenken des Roboterarmes (1) Sensoren zur Erfassung der Gelenkstellung in einem in der Programmsteuerung definierten Werkzeug-Koordinatensystem angeordnet sind und wobei der Roboterarm (1) mittels der Programmsteuerung in eine Arbeitsposition für den chirurgischen Eingriff bewegbar ist, **dadurch gekennzeichnet, dass** die Sensoreinrichtung aus einer Messanordnung (4') mit Datenfernübertragung besteht, die einen stationären Empfänger (7) und einen Taststift (8) mit Sender (9) aufweist,
wobei der Sender (9) mehrere Signalgeber (11) in einer fest vorgegebenen räumlichen Zuordnung aufweist, die von dem Empfänger (7) aufgenommene Ortungssignale abgeben, und wobei
a) an der Montageplatte (2) ein zweiter Sender (10) angeordnet ist, der ebenfalls mehrere Signalgeber (11) in einer fest vorgegebenen räumlichen Zuordnung aufweist, und der Rechner (12) die aus den Ortungssignalen ermittelten Raumkoordinaten des Taststiftes (8) mit den ebenfalls aus den Ortungssignalen ermittelten Raumkoordinaten der Montageplatte (2) in die Koordinaten des Werkzeug-Koordinatensystems transformiert oder
b) an der Montageplatte (2) mindestens ein Referenzpunkt vorgesehen ist, der ebenso wie die Referenzpunkte am Knochen des Patienten mit Hilfe des Taststiftes (8) abtastbar ist, und der Rechner die aus den Ortungssignalen ermittelten Raumkoordinaten des Taststiftes (8) mit den ebenfalls aus den Ortungssignalen ermittelten Raumkoordinaten der Montageplatte (2) in die Koordinaten des Werkzeug-Koordinatensystems transformiert.

4. Vorrichtung nach Anspruch 3, **gekennzeichnet durch** eine zusätzliche Überwachungseinrichtung zur Erfassung der räumlichen Position des Patienten während der Operation mit mindestens einem Referenzstift, der vor dem chirurgischen Eingriff an dem betreffenden Knochen des Patienten befestigbar ist und mit einem dritten Sensor ausgerüstet ist, wobei der ebenfalls mit mehreren Signalgebern ausgerüstete Sensor des Referenzstiftes von dem Empfänger aufgenommene Ortungssignale abgibt, die im Rechner verarbeitbar sind, so dass **durch** die Programmsteuerung eine an die Positionsänderung des Patienten angepasste Korrektur der Arbeitsposition des Roboterarmes (1) erfolgt.

5. Vorrichtung nach einem der Ansprüche 1 bis 4, **dadurch gekennzeichnet, dass** das Werkzeug (3) auf einem an der Montageplatte (2) befestigten Schlitten axial beweglich geführt ist.

6. Vorrichtung nach Anspruch 5, **dadurch gekennzeichnet, dass** der Schlitten (6) in einem festen Winkel, bevorzugt orthogonal zur Montageplatte (2) angeordnet ist.

## Claims

1. Device for positioning and guidance of a surgical instrument during orthopaedic operations with
an industrial robot, having a program-controlled, multi-jointed robot arm with a mounting plate at its end,
an instrument for the surgical procedure held on the mounting plate,
a program control system with computer,
a sensor arrangement for the sensing of reference points on the bones of the patient,
such that sensors are arranged in the joints of the robot arm for determining the joint positions in an instrument coordinate system defined in the program control system and such that the robot arm can be moved by means of the program control system into a working position for the surgical procedure, **characterised in that** the sensor arrangement comprises a multi-jointed sensor arm (4), this being executed as a passive, manually-guided measuring instrument, has sensors for detecting the joint positions and is also available to the surgeon during the operation,
wherein through movement of the sensor arm (4) the reference points on the patient's bone can be sensed, while the robot arm (1) is positioned in a home position defined by the robot control system,
wherein the sensor arm (4) either
a) is affixed to the mounting plate (2) of the robot arm (1) and the computer (12) determines the working position of the robot arm (1) for the surgical procedure from the positional details supplied by the sensors of the sensor arm (4) and the positional details of the robot arm sensors measured in the instrument coordinate system or
b) the sensor arm (4) is so positioned stationary that both the patient's bone to be sensed and the mounting plate (2) of the robot arm (1) positioned in a home position lie within the working space measurable with the sensor arm (4), and the computer (12) transforms the coordinates of the reference points determined with the sensor arm (4) in a spatially fixed three-dimensional coordinate system into the coordinates of the instrument coordinate system.

2. Device according to Claim 1, **characterised in that** it has an additional monitoring arrangement to detect the spatial position of the patient during the operation with a camera, which detects the positions of marking pegs inserted in the relevant bone before the operation, the coordinates of which pegs can be sensed with aid of the sensor arm (4) on setting-up the working position of the robot arm (1), such that positional changes in the marking pegs detected by the camera are processed in the computer and a correction is made to the working position of the robot arm (1) by means of the program control system.

3. Device for positioning and guidance of a surgical instrument during orthopaedic operations with
an industrial robot, having a program-controlled, multi-jointed robot arm with a mounting plate at its end,
an instrument for the surgical procedure held on the mounting plate,
a program control system with computer,
a sensor arrangement for the sensing of reference points on the bones of the patient,
such that sensors are arranged in the joints of the robot arm for determining the joint positions in an instrument coordinate system defined in the program control system and such that the robot arm can be moved by means of the program control system into a working position for the surgical procedure, **characterised in that** the sensor arrangement comprises a measuring arrangement (4') with remote data transfer, having a stationary receiver (7), a sensing probe (8) with a first transmitter (9)
wherein the transmitter (9) has multiple signal generators (11) in a rigidly defined spatial arrangement, that emit positioning signals received by the receiver (7), and wherein
a) a second transmitter (10) is arranged on the mounting plate (2), wherein the second transmitter (10) also has multiple signal generators (11) in a rigidly defined spatial arrangement and the computer (12) transforms the spatial coordinates of the sensing probe (8) determined from the positioning signals with the spatial coordinates of the mounting plate, also determined from the positioning signals into the coordinates of the instrument coordinate system or
b) wherein the mounting plate (2) has at least one reference point, which can be sensed as well as the reference points on the patient's bone with the aid of the sensing probe (8) and the computer (12) transforms the spatial coordinates of the sensing probe (8) determined from the positioning signals with the spatial coordinates of the mounting plate, also determined from the positioning signals into the coordinates of the instrument coordinate system.

4. Device according to Claim 3, **characterised in that** it has an additional monitoring arrangement to detect the spatial position of the patient during the operation with at least one reference peg, which can be affixed to the patient's relevant bone before the surgical procedure and is equipped with a third sensor, such that the transmitter of the reference peg, which is also equipped with multiple signal generators, emits positioning signals that are received by the receiver, which are processable in the computer (12), such that a correction of the working position of the robot arm (1) adapted to positional changes of the patient is undertaken by the program control system.

5. Device according to one of the Claims 1 to 4, **characterised in that** the instrument (3) is guided on a carriage affixed to the mounting plate (2), so that it is moveable axially.

6. Device according to Claim 5, **characterised in that** the carriage (6) is arranged at fixed angle, preferably orthogonal to the mounting plate (2).

## Revendications

1. Dispositif permettant de positionner et de diriger un instrument chirurgical lors d'interventions orthopédiques, comportant
un robot industriel équipé d'un bras robotisé (1) à plusieurs articulations contrôlé par programme, avec plaque finale de montage (2),
un instrument (3) fixé sur la plaque de montage (2) et destiné à l'intervention chirurgicale,
une commande par programme avec ordinateur (12) et
un équipement sensoriel destiné au balayage de points de référence prévus sur des os du patient,
des capteurs de détection de la position des articulations au sein d'un système de coordonnées de l'instrument défini dans la commande par programme étant disposés dans les articulations du bras robotisé (1), le bras robotisé (1) étant déplaçable à l'aide de la commande par programme en une position de travail destinée à l'intervention chirurgicale, **caractérisé en ce que** l'équipement sensoriel est constitué d'un bras palpeur (4) à plusieurs articulations, exécuté comme instrument de mesure passif, guidable manuellement, qu'il comprend des capteurs de détection de la position des articulations et qu'il est mis à la disposition du chirurgien également durant l'intervention, les points de référence sur l'os du patient pouvant être balayés par mouvement du bras palpeur (4),
alors que le bras robotisé (1) se trouve dans une position initiale prédéterminée par la commande du robot et approchée auparavant,
le bras palpeur (4) étant soit
a) fixé sur la plaque finale de montage (2) du bras robotisé (1), l'ordinateur (12) déterminant la position de travail du bras robotisé (1) en vue de l'intervention chirurgicale, à partir des indications de position fournies par les capteurs du bras palpeur (4) et des indications de position des capteurs du bras robotisé mesurées dans le système de coordonnées de l'instrument, soit
b) que le bras palpeur (4) est disposé de manière stationnaire; de sorte qu'aussi bien l'os devant être palpé que la plaque de montage (2) du bras robotisé (1) déplacé dans sa position initiale se trouvent dans l'espace de travail à balayer par le bras palpeur (4), l'ordinateur (12) transformant les coordonnées des points de référence détectés à l'aide du bras palpeur (4) dans un système de coordonnées tridimensionnel stabilisé en des coordonnées du système de coordonnées de l'instrument.

2. Dispositif selon la revendication 1, **caractérisé par** un équipement complémentaire de surveillance destiné à la détection de la position spatiale du patient durant l'opération à l'aide d'une caméra détectant la position de pions de marquage placés dans l'os en cause avant l'intervention chirurgicale, et dont les coordonnées peuvent être balayées à l'aide du bras palpeur (4) en vue de l'aménagement de la position de travail du bras robotisé (1), des changements de position des pions de marquage captés par la caméra étant traités dans l'ordinateur, une correction de la position de travail du bras robotisé (1) étant ensuite effectuée par la commande par programme.

3. Dispositif permettant de positionner et de diriger un instrument chirurgical lors d'interventions orthopédiques, comportant
un robot industriel équipé d'un bras robotisé (1) à plusieurs articulations contrôlé par programme, avec plaque finale de montage (2),
un instrument (3) fixé sur la plaque de montage (2) et destiné à l'intervention chirurgicale,
une commande par programme avec ordinateur (12) et
un équipement sensoriel destiné au balayage de points de référence prévus sur des os du patient,
des capteurs de détection de la position des articulations au sein d'un système de coordonnées de l'instrument défini dans la commande par programme étant disposés dans les articulations du bras robotisé (1), le bras robotisé (1) étant déplaçable à l'aide de la commande par programme en une position de travail destinée à l'intervention chirurgicale, **caractérisé en ce que** l'équipement sensoriel est constitué d'un montage de mesure (4') avec télétransmission de données, comprenant un récepteur (7) stationnaire et un palpeur (8) avec émetteur (9),
l'émetteur (9) étant constitué de plusieurs transmetteurs de signaux (11) agencés dans un espace prédéfini, émettant des signaux de localisation captés par le récepteur (7), et
a) un deuxième émetteur (10) étant disposé sur la plaque de montage (2), lequel est également constitué de plusieurs transmetteurs de signaux (11) agencés dans un espace prédéfini, l'ordinateur (12) transformant les coordonnées spatiales déterminées par le palpeur (8) et venant des signaux de localisation, de même que les coordonnées spatiales déterminées par la plaque de montage (2) et venant également des signaux de localisation en les coordonnées du système de coordonnées de l'instrument, ou
b) un point de référence au moins étant prévu sur la plaque de montage (2), lequel point de référence peut être balayé à l'aide du palpeur (8), tout comme les points de référence marqués sur l'os du patient, l'ordinateur transformant les coordonnées spatiales déterminées par le palpeur (8) et venant des signaux de localisation, de même que les coordonnées spatiales déterminées par la plaque de montage (2) et venant également des signaux de localisation en les coordonnées du système de coordonnées de l'instrument.

4. Dispositif selon la revendication 3, **caractérisé par** un équipement complémentaire de surveillance destiné à la détection de la position spatiale du patient durant l'opération à l'aide d'un palpeur de référence au moins, lequel palpeur peut être fixé avant l'intervention chirurgicale sur l'os en cause du patient et est équipé d'un troisième capteur, le capteur du palpeur de référence qui est également équipé de plusieurs transmetteurs de signaux émettant des signaux de localisation captés par le récepteur, ces signaux pouvant être traités dans l'ordinateur, de sorte que la commande par programme engendre une correction de la position de travail du bras robotisé (1) adaptée au changement de position du patient.

5. Dispositif selon l'une quelconque des revendications 1 à 4, **caractérisé en ce que** l'instrument (3) est installé sur un coulisseau fixé sur la plaque de montage (2) et guidé de manière axiale.

6. Dispositif selon la revendication 5, **caractérisé en ce que** le coulisseau (6) est disposé dans un angle fixe par rapport à la plaque de montage (2) avantageusement dans un angle orthogonal.
